# EUROPEAN PATENT SPECIFICATION

(11) **EP 0 438 234 B1**
(45) Date of publication and mention of the grant of the patent: **12.04.1995**
(21) Application number: 91300196.2
(22) Date of filing: 11.01.1991
(51) Int. Cl.: A61K 38/00, A61K 35/16

(54) **Antithrombin solution and use thereof**
Antithrombin-Lösung und ihre Verwendung
Solution d'antithrombine et son utilisation

(30) Priority: 19.01.1990 JP 8230/90
(43) Date of publication of application: 24.07.1991
(73) Proprietor: Kita, Kiyoshi, Shibuyaku Tokyo (JP)
(72) Inventor: Kita, Kiyoshi, Shibuyaku Tokyo (JP)
(74) Representative: Pearce, Anthony Richmond

(56) References cited:
- EP-A- 0 255 651
- EP-A- 0 315 968

## Description

Most of the joints in the body represent synovial joints which have four distinguishing features: (1)the articulating surfaces of the bones are covered with hyaline cartilage called articulating cartilage; (2)the joint is enclosed by an articular capsule or joint capsule; (3)the inner layer of the joint capsule is lined with a thin vascular synovial membrane; and (4)the synovial membrane encloses a cavity into which a synovial fluid is secreted.

The causes of arthritis include trauma to a joint, bacterial infection and metabolic disorders.

Rheumatoid arthritis, the most important clinically, begins with inflammation of the synovial membrane; the inflammed synovial membrane produces an abnormal tissue known as pannus which grows over the articular cartilage. The articular cartilage is distorted and is occasionally destroyed. Current medications for arthritis include sodium aurothiomalate, penicillamine, antipyretic antiphlogistics such as aspirin and indomethacin, and corticosteroids. The severe pain caused by active arthritis can be temporarily relieved through the administration of hydrocortisone which shows a strong anti-inflammatory effect and also makes hyaluronic acid (hereinafter referred to as HA) in normal highly polymerized states. Corticosteroids are prescribed even though it is common knowledge for doctors to be cautious of the side effects like osteoporosis from a long-term prescription of corticosteroids.

The volume of abnormal HA in the synovial fluid increases in cases of infectious and rheumatoid arthritis. This abnormal HA is not properly polymerized and therefore can not maintain or improve joint function. If a properly polymerized HA could be introduced into the joint capsule, the treatment would be much more effective. From that point of view, a local injection of HA has recently been made available as an injection into the synovial cavity of osteoarthritis.

In the wound healing process following surgical operations beginning with the dissection of the skin, ineffective adhesion due to scars on each surgically traumatized organ may result and cause pain, mechanical ileus, functional disturbance, motor disturbance or orthopaedic impairment at the surgical region. These complications occur with the following steps: (1)surgical trauma to the organs; (2) inflammatory exudates, especially fibrin, are secreted on those tissues; (3)fibrino-fibrous inflammation on the tissues: (4) the fibrous strands on the organs contract; and (5) the complications occur.

Balazs et al.(a) infra, reported in 1973 that HA inhibits cell migration and multiplication of certain cells in vitro. And it is speculated that HA influences the invasion and activity of cells participating in the acute and chronic inflammatory process and it is thought to prevent fibrous tissue formation. However, it does not effectively suppress the formation of fibrin which is converted from excessive fibrinogen under a hypercoagulated condition.

At the same time, there are some reports that HA has a procoagulant activity. If this is true, the fibrin clot formation is to be promoted in areas having fibrinous inflammation like arthritis which may result in more severe ankylosis. Biological activities of implanted HA in the body are not well defined presently. It has not been proven that HA has an anti-inflammatory effect in biological inflammatory processes. It may cease the inflammation of osteoarthritis by improving the cushion effect and mechanical separation of tissues in the joint, not by relating to theoretical inflammation processes directly.

Even if HA indirectly affects the inflammation process, it does not effectively inhibit fibrous tissue formation, pannus nor ankylosis.

Implanted HA in the joint would temporarily be a physical barrier, not a biological inhibitor, for the invasion of exudates such as fibrin and inflammatory cells. Even though, it is considered that the implanted HA would mix with various inflammatory exudates under the mobility of the joint, and would be unable to inhibit the cell migration and fibrin clot formation in the synovial cavity.

LeBoeuf et al (b) infra, reported in 1986 that human fibrinogen specifically binds hyaluronic acid in vitro, and suggested a close interaction between fibrin and HA. Moreover, excessive fibrinogen is more quickly converted to fibrin under the conditions of a depressed activity of plasminogen activator or lowered antithrombin concentrations which occur within the inflamed area.

The formation of the fibrous tissue within the articular capsule is a result of an ineffective anticoagulation process within an inflamed synovial joint. The subsequent lysis of the fibrinous tissue is also hindered following the formation of the fibrous tissue, whereat the synovial fluid becomes less viscous and elastic. Scars which may cause pain and ankylosis are formed whereat the fibrin is replaced by collagenous tissue following the invasion of mesenchymal cells such as fibroblasts.

In one aspect, the present invention resides in the use of an antithrombin solution for the manufacture of a medicament for injection into the synovial cavity of a patient with active arthritis, wherein the antithrombin solution comprises antithrombin of at least 0.5 U/ml and not more than 250 U/ml dissolved in a physiological buffer solution.

Preferably, in said one aspect, the antithrombin solution further comprises a substance selected from sodium hyaluronate and sodium chondroitin sulfate. The substance is preferably sodium hyaluronate which preferably has an average molecular weight between 350,000 and 8,000,000 and is present in the solution in an amount of 0.1 to 3% by weight.

In another aspect, the present invention resides in the use of an antithrombin solution for the manufacture of a medicament for coating exposed tissue of internal surgical regions during an operation beginning with the dissection of the skin to avoid postoperative adhesion of the surgically traumatized organs, wherein the antithrombin solution comprises not more than 250 U/ml of antithrombin dissolved in a physiological buffer solution.

The antithrombin solution, in said other aspect, preferably further comprises a viscous substance selected from sodium hyaluronate, sodium chondroitin sulfate, polyacrylamide, hydroxypropylmethylcellulose and collagen type IV. The use of sodium hyaluronate is preferred, particularly sodium hyaluronate having an average molecular weight of 50,000 to 13,000,000.

In a further aspect, the present invention resides in an antithrombin solution comprising 0.5 U/ml to 250 U/ml of antithrombin, and a substance selected from sodium hyaluronate, sodium chondroitin sulfate, polyacrylamide, hydroxypropylmethylcellulose and collagen type IV in a physiological buffer solution.

The introduction of antithrombin (hereinafter referred to as AT) into the synovial fluid or other intracorporeal regions inactivates thrombin necessary for fibrin formation.

The use of a viscous material, such as a sodium hyaluronate solution, with AT prolongs the effectiveness of the latter as long as possible, wherein it can suppress the local coagulation system at the synovial cavity or other intracorporeal surgical regions.

For the purpose of improved treatment, this invention provides AT in a viscoelastic solution for therapeutic use. This solution utilizes the characteristics of the elasto-viscous and protein binding effects of sodium hyaluronate solution, and a biological anti-coagulation effect of AT-glycoprotein.

The biophysical element, AT, which inhibits thrombin action, theoretically exists in several types, such as, I, II, III, etc. However, only AT-III having a molecular weight between 58,000 and 65,000 has been proven actually to exist. Human AT is a glycoprotein of a molecular weight 58,000 made up of 425 amino acids in a single polypeptide chain crosslinked by three disulfide bridges. That is, it is synthesized in the liver, exists in the blood, and controls the coagulation system. It forms a complex with heparin and shows a strong antithrombin effect. AT-III gradually inhibits the action of thrombin when heparin is absent, and instantaneously neutralizes thrombin when heparin is present. In other words, heparin alone does not cause anti-coagulation, but it accelerates the action of AT-III.

Pharmacokinetic studies have shown AT to have a mean biological half-life of about 3.0 days in blood. The half-life is shortened in presence of heparin.

The quantity of AT in 1ml normal pooled human plasma is conventionally taken as one unit (U). AT's potency assignment was determined from a standard calibrated against a World Health Organization (WHO) AT reference preparation. The concentration of AT in normal human plasma has been estimated at between 0.1 and 0.2 g/l, and thrombin inactivating activity levels are usually expressed either as a percentage of a reference plasma or as the IU number per ml of plasma, one IU per ml being equivalent to 100%.

In order to provide treatment for disseminated intravascular coagulation (D.I.C.) in the past, heparin was mainly administered therewith. However, it has already been shown that heparin does not work effectively when blood levels of AT have dropped. Accordingly, it has been recommended to administer dried concentrated human AT-III, so that the lack of AT can be corrected.

This invention is directed to the local administration of AT into the mammalian intracorporeal regions in order to improve the local hypercoagulability.

Several investigators recently studied the effectiveness of recombinant tissue plasminogen activator (t-PA) and reported that doctors should be cautious of postoperative hemorrhages resulting from the lysis of hemostatic thrombi, when using this strong fibrinolytic agent. On the other hand, AT inhibits fibrin formation and we should be cautious of inhibiting the wound healing process.

A solution having more than about 250 U/ml of AT should be avoided due to a higher tendency for spontaneous hemorrhaging.

The administration of an AT solution to patients having a hemorrhagic tendency or an inhibition to wound healing due to a deficiency of a blood coagulation factor, should be avoided.

HA, a large polysaccaride molecule, is present in nearly all connective tissue matrices of vertebrate organisms. In the human body, it is an important structural element in the skin, subcutaneous and interstitial connective tissues, synovial tissue and fluid, umbilical cord and the vitreous; its brief roles are speculated to be: (1) a vehicle for biophysical and biologically active elements; (2)adhesion to tissues and cells; (3)protection, separation and lubrication of tissues and/or cells; and (4)maintaining an equilibrium of water and sodium chloride within the connective tissues.

The molecular weight of a purified fraction of HA after isolation is known to be within the range of 50,000 to 13,000,000. It has been confirmed that the solution of deproteinized and purified sodium hyaluronate is safe for use in ocular surgery and osteoarthritis. The concentrations of sodium hyaluronate for ocular surgery and osteoarthritis are generally from 0.1 to 3% in weight. Its molecular weight generally ranges from about 500,000 to 5,000,000.

In view of the fact that the sodium hyaluronate solution needs at least three days to dissipate from the joint, this invention utilizes a mixture of AT with a viscous solution, whereas the AT works over an extended period to control the fibrin accumulation in the synovial cavity.

A physiological buffer solution mentioned in this specification includes distilled water with sodium chloride, buffers and/or stabilizers. Buffers are preferably selected from sodium phosphate and dissodium phosphate, and stabilizers for AT are also preferably selected from sodium citrate, amino acetic acid, sodium glutamate, acetyltryptophan and sodium caprylate. These buffers and stabilizers are presently used for the sodium hyaluronate and AT solutions. The above mentioned buffers and/or stabilizers are favorable though this invention is not limited to any specific type of buffer or stabilizer, but rather to any usable solution which qualifies as a buffer/stabilizer.

The sterile final AT solution is used by either injection into the synovial cavity or by coating the tissue of the exposed internal surgical region intraoperatively.

The materials such as collagen type-IV, chondroitin sulfate (a molecular weight between 5,000 to 100,000), polyacrylamide (a molecular weight cf about 1,000.000) and hydroxypropylmethylcellulose (a molecular weight of about 1,000,000) may be applicable as a substitute of the sodium hyaluronate or it may be combined with the sodium hyaluronate, even though, in this specification, HA is described as the preferred viscous substance due to its favorable elastoviscous and biophysical characteristics.

Artificially synthesized HA and AT would be applicable to the invented method of administration of an AT solution as a substitute of the previously mentioned variety of HA and AT-III compounds.

Without further elaboration, it is believed that one skilled in the art, using the preceding description, can utilize the present invention to its fullest extent. The following possible preferred administration examples for human are, therefore, to be construed as merely illustrative.

### (Possible Method of Administration, Example I)

Two tenths of a milliliter of the following AT solution are injected into the synovial cavity without removal of the synovial fluid from the joint.

Human AT-III having a molecular weight of about 59,000 is dissolved in a 1ml physiological buffer solution to obtain a concentration of 50 U/ml.

### (Possible Method of Administration, Example II)

Two milliliters of the following AT solution are injected into the synovial cavity upon removal of 2ml of the synovial fluid from the joint.

Ten units per milliliter of human AT-III having a molecular weight of about 58,000, and 100mg of sodium hyaluronate having an average molecular weight of about 800,000, are dissolved in a 10ml physiological buffer solution.

### (Possible Method of Administration, Example III)

Two milliliters of the following AT solution are injected into the synovial cavity without removing the synovial fluid from the joint.

Ten units per milliliter of human AT-III having a molecular weight of about 58,000, and 100mg of sodium hyaluronate having an average molecular weight of about 800,000, are dissolved in a 10ml physiological buffer solution.

### (Possible Method of Administration, Example IV)

The tissue of exposed and surgically traumatized abdominal organs is coated with the following AT solution during abdominal surgery, and the surgery is closed without removing the AT solution.

Ten units per milliliter of human AT-III having a molecular weight of about 58,000 and 1g of sodium hyaluronate having an average molecular weight of about 4,000,000 are dissolved in a 100ml physiological buffer solution.

### (Possible Method of Administration Example V)

The tissue of exposed and surgically traumatized abdominal organs is coated with the following AT solution during abdominal surgery, and the surgery was closed without removing the AT solution.

Human AT-III having a molecular weight of about 58,000 is dissolved in a 100ml physiological buffer solution to obtain a concentration of 50 U/ml. Simultaneously, 3g of sodium hyaluronate having an average molecular weight of about 500,000 and 4g of sodium chondroitin sulfate having an average molecular weight of about 25,000 are dissolved in the solution.

The preceding examples of possible method of administration can be repeated with similar success by substituting the generically or specifically described reactants and or operating conditions of this invention for those used in the preceding examples of possible method of administration.

By introducing this new method of adminstration of the AT solution into the synovial cavity of patients with active arthritis, fibrin formation in the synovial cavity can be prevented. In this regard, the invention decreases the complications seen with the excessive use of anti-arthritis drugs such as sodium aurothiomalate, penicillamine, antipyrine, aspirin, indomethacin and corticosteroids.

AT with a concentration from 0.5 to 250 U/ml and sodium hyaluronate having an average molecular weight between 350,000 and 8,000,000 with a concentration from 0.1 to 3% are recommended for effective treatment of the joint.

The average concentration of normal HA in the human synovial cavity is reported to be about 0.15 to 0.4% by Hadler et al.(c) infra, in 1979. A concentration as low as 0.1% HA solution is possible when the HA has a relatively high molecular weight, i.e. greater than 3,000,000, and a concentration as high as 1 to 3% is also possible when the HA has a relatively low molecular weight, i.e. lower than 1,000,000.

By introducing this invention not only to the joint but also to the intracorporeal surgeries(such as intraperitoneal, cesarean section, heart, lung, visceral, intracranial, ear, nose, throat and flexor tendon surgeries) postoperative pain, mechanical ileus, functional disturbance, motor disturbance or orthopaedic impairments due to scars caused by the fibrin clot formations in the surgical region can be prevented.

This invention is also useful for coating the exposed internal surgical region with the AT solution during the operation, and subsequently closing the surgery without removing the AT solution from the surgical region.

When using sodium hyaluronate of relatively low (as low as 50,000 to 1,000,000) or high (as high as 4,000,000 to 13,000,000) average molecular weight, the increased or decreased concentration of sodium hyaluronate would be possible in order to maintain an effective viscosity of the AT solution.

### References

(a) Balazs et al., in Biology of the Fibroplast, pp. 237-252,
   Academic Press, London, 1973
   Darzynkiewicz et al., Extl.Cell Res., 66 (1971) pps. 113-123
   A. Balazs, U.S. Pat. 4141973
(b) Robert D. Leboeuf et al., in The Journal of Biological Chemistry, Vol. 261, No. 27, pp. 12586-12592, 1986, U.S.A
(c) N.M. Hadler et al., Glycoconjugate Research, 1,525 (1979)
   A. Ferbert et al., BWtPA Acute Embolic Apoplexy Investigation Group, USA and Germany, Medical Tribune, Japan, 1989, The 1st. Internationl Apoplexy Congress.
   U. Abildgaard, Antithrombin Deficiency, KabiVitrum AB, Sweden, 1988
   Antithrombin, Kabi Plasma Products, Sweden, 1989
   K. Horie, Fragrance Journal, No.9, pp. 160-166, 1988, Japan
   E.F. Mammen et al., Biologia & Clinica Hematologica, 9, Supl.1, 69-73, 1987

## Claims

1. The use of an antithrombin solution for the manufacture of a medicament for injection into the synovial cavity of a patient with active arthritis, wherein the antithrombin solution comprises antithrombin of at least 0.5 U/ml and not more than 250 U/ml dissolved in a physiological buffer solution.

2. The use as claimed in claim 1, wherein the antithrombin solution further comprises 0.1 to 3% by weight of sodium hyaluronate having an average molecular weight of between 350,000 and 8,000,000.

3. The use as claimed in claim 1, wherein the antithrombin solution further comprises a substance selected from sodium hyaluronate and sodium chondroitin sulfate.

4. The use of an antithrombin solution for the manufacture of a medicament for coating exposed tissue of internal surgical regions during an operation beginning with the dissection of the skin to avoid postoperative adhesion of the surgically traumatized organs, wherein the antithrombin solution comprises at least 0.5 U/ml and not more than 250 U/ml of antithrombin dissolved in a physiological buffer solution.

5. The use as claimed in claim 4, wherein the antithrombin solution further comprises 0.1 to 3% by weight of sodium hyaluronate having an average molecular weight within the range of 50,000 to 13,000,000.

6. The use as claimed in claim 4, wherein the antithrombin solution further comprises a viscous substance selected from sodium hyaluronate, sodium chondroitin sulfate, polyacrylamide, hydroxypropylmethylcellulose and collagen type IV.

7. An antithrombin solution comprising 0.5 U/ml to 250 U/ml of antithrombin, and a substance selected from sodium hyaluronate, sodium chondroitin sulfate, polyacrylamide, hydroxypropylmethylcellulose and collagen type IV in a physiological buffer solution.

8. An antithrombin solution as claimed in claim 7, containing 0.1 to 3% by weight of sodium hyaluronate having an average molecular weight within the range of 50,000 to 13,000,000.

9. An antithrombin solution as claimed in claim 8, wherein the sodium hyaluronate has an average molecular weight between 350,000 and 8,000,000.

## Patentansprüche

1. Verwendung einer Antithrombin-Lösung für die Herstellung eines Medikaments zur Injektion in die Synovialhöhle eines Patienten mit aktiver Arthritis, wobei die Antithrombin-Lösung mindestens 0,5 E/ml und nicht mehr als 250 E/ml in einer physiologischen Pufferlösung gelöstes Antithrombin aufweist.

2. Verwendung nach Anspruch 1, wobei die Antithrombin-Lösung ferner 0,1 bis 3 Gew.-% Natriumhyaluronat mit einem mittleren Molekulargewicht zwischen 350 000 und 8 000 000 aufweist.

3. Verwendung nach Anspruch 1, wobei die Antithrombin-Lösung ferner eine unter Natriumhyaluronat und Natriumchondroitinsulfat ausgewählte Substanz aufweist.

4. Verwendung einer Antithrombin-Lösung zur Herstellung eines Medikaments zum Überziehen freiliegenden Gewebes innerer Operationsbereiche während einer Operation, die mit der Dissektion der Haut beginnt, um eine postoperative Adhäsion der chirurgisch traumatisierten Organe zu vermeiden, wobei die Antithrombin-Lösung mindestens 0,5 E/ml und nicht mehr als 250 E/ml in einer physiologischen Pufferlösung gelöstes Antithrombin aufweist.

5. Verwendung nach Anspruch 4, wobei die Antithrombin-Lösung ferner 0,1 bis 3 Gew.-% Natriumhyaluronat mit einem mittleren Molekulargewicht zwischen 50 000 und 13 000 000 aufweist.

6. Verwendung nach Anspruch 4, wobei die Antithrombin-Lösung ferner eine unter Natriumhyaluronat, Natriumchondroitinsulfat, Polyacrylamid, Hydoxypropylmethylcellulose und Collagen Typ IV ausgewählte viskose Substanz aufweist.

7. Antithrombin-Lösung, die 0,5 E/ml bis 250 E/ml Antithrombin und eine unter Natriumhyaluronat, Natriumchondroitinsulfat, Polyacrylamid, Hydoxypropylmethylcellulose und Collagen Typ IV ausgewählte Substanz in einer physiologischen Pufferlösung aufweist.

8. Antithrombin-Lösung nach Anspruch 7, die 0,1 bis 3 Gew.-% Natriumhyaluronat mit einem mittleren Molekulargewicht im Bereich von 50 000 bis 13 000 000 enthält.

9. Antithrombin-Lösung nach Anspruch 8, wobei das Natriumhyaluronat ein mittleres Molekulargewicht zwischen 350 000 und 8 000 000 aufweist.

## Revendications

1. Utilisation d'une solution d'antithrombine pour la préparation d'un médicament injectable dans la cavité synoviale d'un patient souffrant d'arthrite aiguë, la solution d'antithrombine comprenant au moins 0,5 U/ml et pas plus de 250 U/ml d'antithrombine dissoute dans une solution physiologique tampon.

2. Utilisation selon la revendication 1, dans laquelle la solution d'antithrombine comprend en outre 0,1 à 3% en poids d'hyaluronate de sodium d'un poids moléculaire moyen compris entre 350.000 et 8.000.000.

3. Utilisation selon la revendication 1, dans laquelle la solution d'antithrombine comprend en outre une substance sélectionnée parmi l'hyaluronate de sodium et le sulfate chondroïtique de sodium.

4. Utilisation d'une solution d'antithrombine pour la préparation d'un médicament pour enduire les tissus exposés de régions chirurgicales internes au cours de l'intervention, débutant par la dissection de la peau, pour empêcher une adhésion postopératoire aux organes traumatisés par la chirurgie, la solution d'antithrombine comprenant au moins 0,5 U/ml et pas plus de 250 U/ml d'antithrombine dissoute dans une solution physiologique tampon.

5. Utilisation selon la revendication 4, dans laquelle la solution d'antithrombine comprend en outre 0,1 à 3% en poids d'hyaluronate de sodium, d'un poids moléculaire moyen compris entre 50.000 et 13.000.000.

6. Utilisation selon la revendication 4, dans laquelle la solution d'antithrombine comprend en outre une substance visqueuse sélectionnée parmi l'hyaluronate de sodium, le sulfate chondroïtique de sodium, le polyacrylamide, l'hydroxypropylméthylcellulose et le collagène de type IV.

7. Solution d'antithrombine comprenant entre 0,5 U/ml et 250 U/ml d'antithrombine, ainsi qu'une substance sélectionnée parmi l'hyaluronate de sodium, le sulfate chondroïtique de sodium, le polyacrylamide, l'hydroxypropylméthylcellulose et le collagène de type IV dans une solution physiologique tampon.

8. Solution d'antithrombine selon la revendication 7, contenant entre 0,1 et 3% en poids d'hyaluronate de sodium d'un poids moléculaire moyen compris entre 50.000 et 13.000.000.

9. Solution d'antithrombine selon la revendication 8, dans laquelle l'hyaluronate de sodium a un poids moléculaire moyen compris entre 350.000 et 8.000.000.
